# EUROPÄISCHE PATENTANMELDUNG

(11) **EP 1 598 351 A1**
(43) Veröffentlichungstag der Anmeldung: **23.11.2005**
(21) Anmeldenummer: 05003841.3
(22) Anmeldetag: 25.07.2000
(51) Int. Cl.: C07D 409/12, A01N 43/66, A01N 43/54

(54) **Substituierte N-Pyrimidinyl-N'-thienyl(amino)sulfonylharnstoffe als Herbizide**

(30) Priorität: 06.08.1999 DE 19937118
(62) Teilanmeldung aus: 00958292.5
(71) Anmelder: Bayer CropScience AG, 40789 Monheim (DE)
(72) Erfinder: Gesing, Ernst Rudolf F., 40699 Erkrath (DE); Kluth, Joachim, 40764 Langenfeld (DE); Müller, Klaus-Helmut, 40593 Düsseldorf (DE); Drewes, Mark Wilhelm, 40764 Langenfeld (DE); Dahmen, Peter, 41470 Neuss (DE); Feucht, Dieter, 65760 Eschborn (DE); Pontzen, Rolf, 42799 Leichlingen (DE)
(74) Vertreter: Schwenk, Norbert

(57) **Zusammenfassung**

Die Erfindung betrifft neue substituierte Thienyl(amino)sulfonylharnstoffe, der allgemeinen Formel (I), 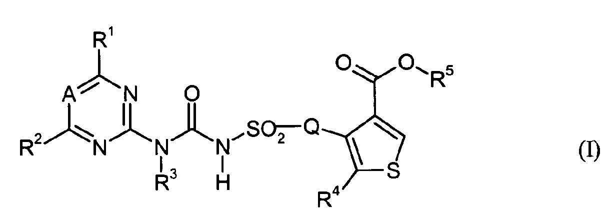 in welcher A, Q, R¹, R², R³, R⁴ und R⁵ die in der Beschreibung angegebene Bedeutung haben,

Verfahren zu ihrer Herstellung und ihre Verwendung als Herbizide.

## Beschreibung

Die Erfindung betrifft neue substituierte Thienyl(amino)sulfonylharnstoffe, Verfahren zu ihrer Herstellung und ihre Verwendung als Herbizide.

Es ist bereits bekannt, daß bestimmte substituierte Thienylsulfonylharnstoffe herbizide Eigenschaften aufweisen (vgl. EP-A-30142 / US-A-4481029 / US-A-4599103 / US-A-4701535, EP-A-97122 / US-A-4549898, EP-A-207609 / US-A-4668281). Die herbizide Wirksamkeit dieser bekannten Verbindungen ist jedoch nicht in allen Belangen zufriedenstellend.

Es wurden nun die neuen substituierten Thienyl(amino)sulfonylharnstoffe der allgemeinen Formel (I) 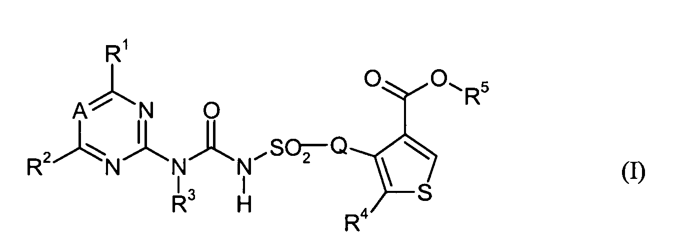 in welcher
- A: für Stickstoff oder eine CH-Gruppierung steht,
- Q: für eine Einfachbindung oder für NH steht,
- R¹: für Wasserstoff, Halogen oder jeweils gegebenenfalls substituiertes Alkyl, Alkoxy, Alkylthio, Alkylamino, Dialkylamino, Aryloxy oder Heterocyclyloxy steht,
- R²: für Wasserstoff, Halogen oder jeweils gegebenenfalls substituiertes Alkyl, Alkoxy, Alkylthio, Alkylamino, Dialkylamino, Aryloxy oder Heterocyclyloxy steht,
- R³: für Wasserstoff oder gegebenenfalls substituiertes Alkyl steht,
- R⁴: für Halogen oder gegebenenfalls substituiertes Alkyl steht und - für den Fall, daß Q für NH steht - auch für Wasserstoff steht, und
- R⁵: für Wasserstoff oder jeweils gegebenenfalls substituiertes Alkyl, Alkenyl, Alkinyl, Cycloalkyl, Cycloalkylalkyl oder Heterocyclyl steht,
sowie Salze von Verbindungen der Formel (I) gefunden.

Gesättigte oder ungesättigte Kohlenwasserstoffgruppierungen, wie Alkyl, Alkenyl oder Alkinyl, sind - auch in Verknüpfungen mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Bevorzugte Substituenten bzw. Bereiche der in den oben und nachstehend aufgeführten Formeln vorhandenen Reste werden im folgenden definiert.
- A: steht bevorzugt für Stickstoff oder eine CH-Gruppierung.
- Q: steht bevorzugt für eine Einfachbindung oder für NH.
- R¹: steht bevorzugt für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy.
- R²: steht bevorzugt für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy.
- R³: steht bevorzugt für Wasserstoff oder gegebenenfalls durch C₁-C₄-Alkoxy, C₁-C₄-Alkyl-carbonyl oder C₁-C₄-Alkoxy-carbonyl substituiertes Alkyl mit 1 bis 4 Kohlenstoffatomen.
- R⁴: steht bevorzugt für gegebenenfalls durch Cyano, Halogen oder C₁-C₄-Alkoxy substituiertes Alkyl mit 1 bis 6 Kohlenstoffatomen steht und - für den Fall, daß Q für NH steht - auch für Wasserstoff.
- R⁵: steht bevorzugt für Wasserstoff, für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Cyano, Halogen, C₁-C₄-Alkyl oder C₁-C₄-Alkoxy substituiertes Oxetanyl, Furyl oder Tetrahydrofuryl.
- A: steht besonders bevorzugt für Stickstoff oder eine CH-Gruppierung.
- Q: steht besonders bevorzugt für eine Einfachbindung oder für NH.
- R¹: steht besonders bevorzugt für Wasserstoff, Fluor, Chlor, Brom, Iod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino.
- R²: steht besonders bevorzugt für Fluor, Chlor, Brom, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino.
- R³: steht besonders bevorzugt für Wasserstoff oder für jeweils gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl.
- R⁴: steht besonders bevorzugt für jeweils gegebenenfalls durch Cyano, Fluor, Chlor, Methoxy oder Ethoxy substituiertes Methyl, Ethyl, n- oder i-Propyl, n-, i-, s- oder t-Butyl.
- R⁵: steht besonders bevorzugt für Wasserstoff, für jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl.
- A: steht ganz besonders bevorzugt für Stickstoff oder eine CH-Gruppierung.
- Q: steht ganz besonders bevorzugt für eine Einfachbindung oder für NH.
- R¹: steht ganz besonders bevorzugt für Wasserstoff, Fluor, Chlor, Brom, für jeweils gegebenenfalls durch Fluor, Chlor, Methoxy oder Ethoxy substituiertes Methyl, Ethyl, Methoxy, Ethoxy, Methylthio, Ethylthio, Methylamino, Ethylamino, oder für Dimethylamino.
- R²: steht ganz besonders bevorzugt für Fluor, Chlor, Brom, für jeweils gegebenenfalls durch Fluor, Chlor, Methoxy oder Ethoxy substituiertes Methyl, Ethyl, Methoxy, Ethoxy, Methylthio, Ethylthio, Methylamino oder Ethylamino, oder für Dimethylamino.
- R³: steht ganz besonders bevorzugt für Wasserstoff oder Methyl.
- R⁴: steht ganz besonders bevorzugt für jeweils gegebenenfalls durch Fluor oder Chlor substituiertes Methyl, Ethyl, n- oder i-Propyl.
- R⁵: steht ganz besonders bevorzugt für jeweils gegebenenfalls durch Fluor, Chlor, Methoxy oder Ethoxy substituiertes Methyl, Ethyl, n- oder i-Propyl, oder für jeweils gegebenenfalls durch Fluor oder Chlor substituiertes Propenyl oder Propinyl.
- A: steht am meisten bevorzugt für eine CH-Gruppierung.
- R¹ und R²: stehen am meisten bevorzugt für Methoxy.
- R³: steht am meisten bevorzugt für Wasserstoff.

Gegenstand der Erfindung sind vorzugsweise auch die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher A, Q, R¹, R², R³, R⁴ und R⁵ die oben vorzugsweise angegebene Bedeutung haben.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Erfindungsgemäß am meisten bevorzugt sind die Verbindungen, bei denen R¹, R², R³ oder A eine der als am meisten bevorzugt ausgeführte Bedeutung bestehen.

Beispiele für die erfindungsgemäßen Verbindungen der Formel (I) sind in den nachstehenden Gruppen aufgeführt. 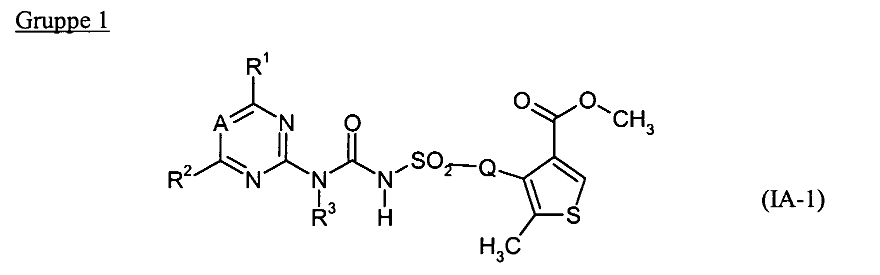 A, Q, R¹, R² und R³ haben darin beispielhaft die nachstehend aufgeführte Bedeutung:

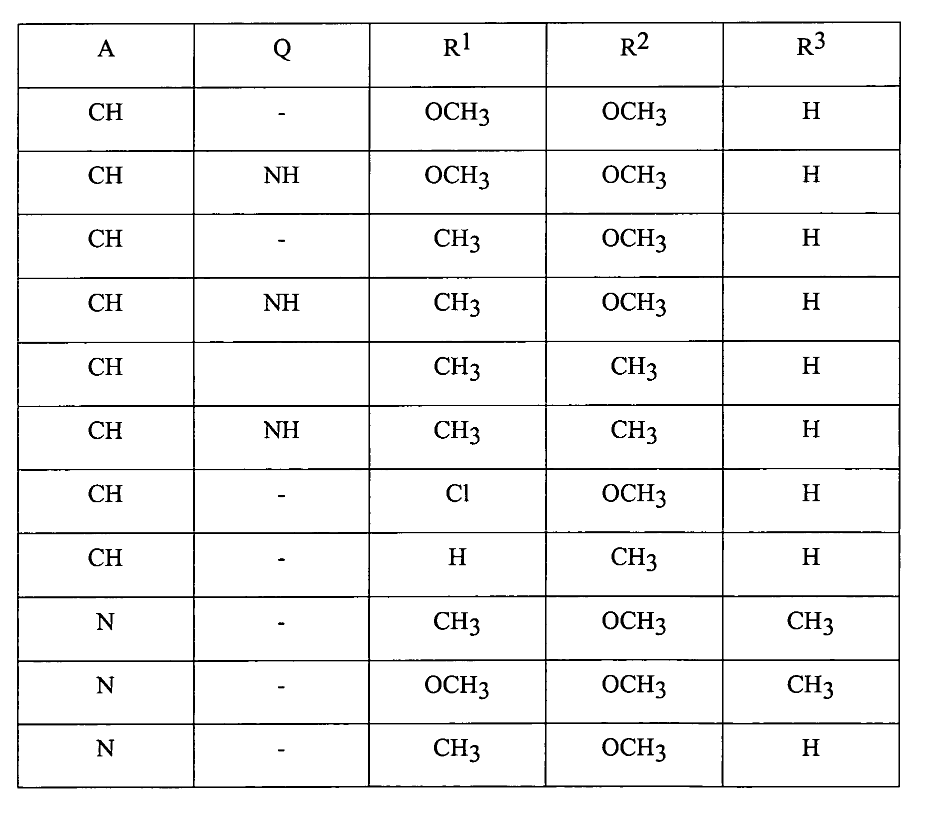

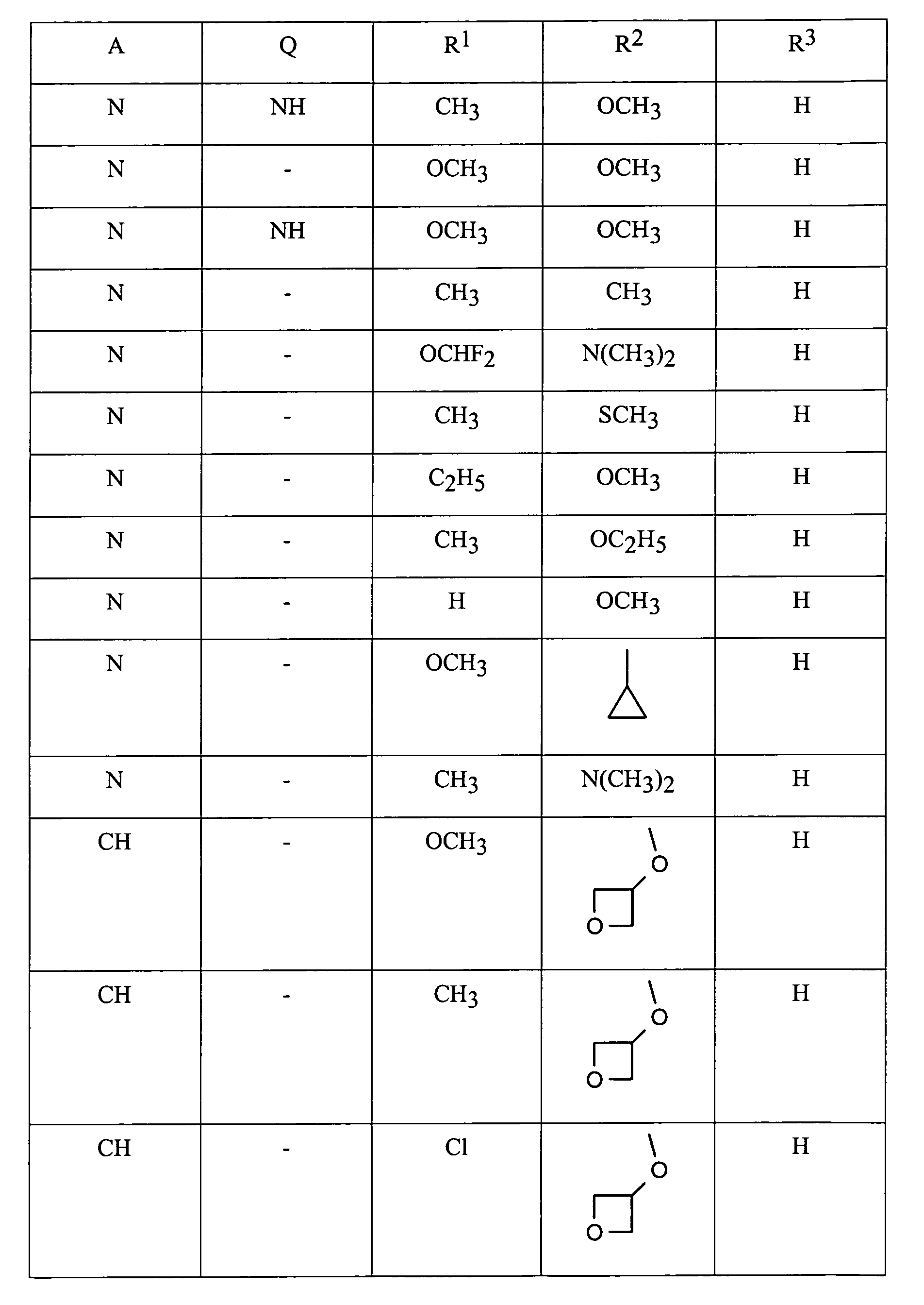

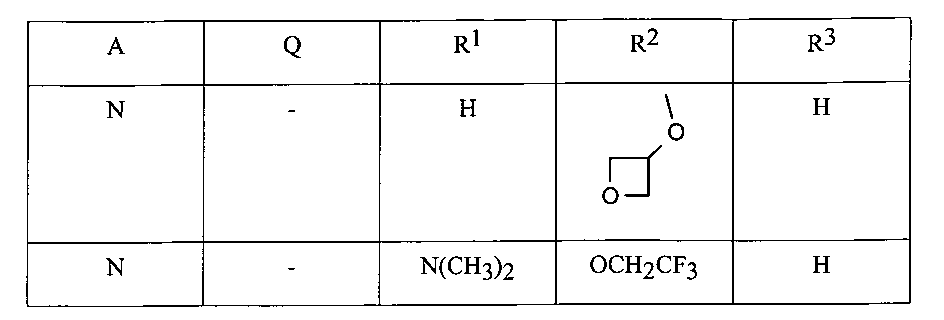

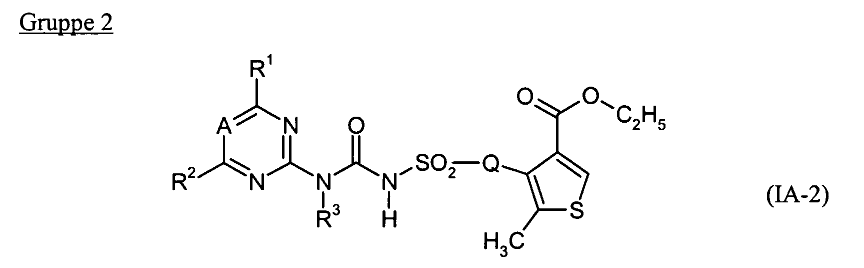

A, Q, R¹, R² und R³ haben hierbei beispielhaft die oben in Gruppe 1 angegebene Bedeutung. 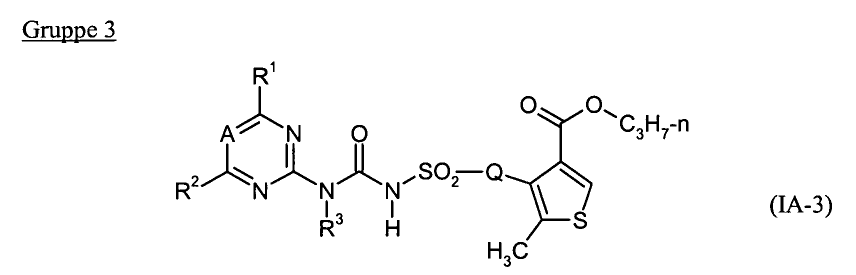 A, Q, R¹, R² und R³ haben hierbei beispielhaft die oben in Gruppe 1 angegebene Bedeutung. 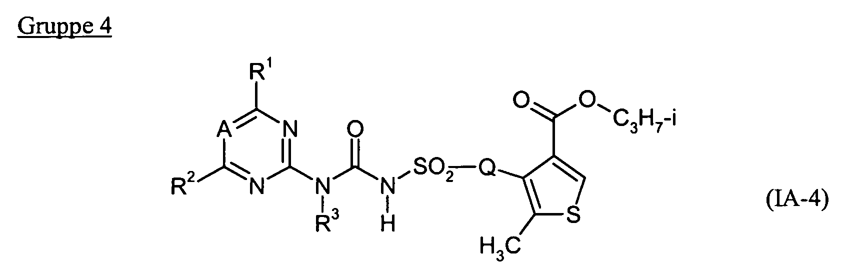 A, Q, R¹, R² und R³ haben hierbei beispielhaft die oben in Gruppe 1 angegebene Bedeutung. 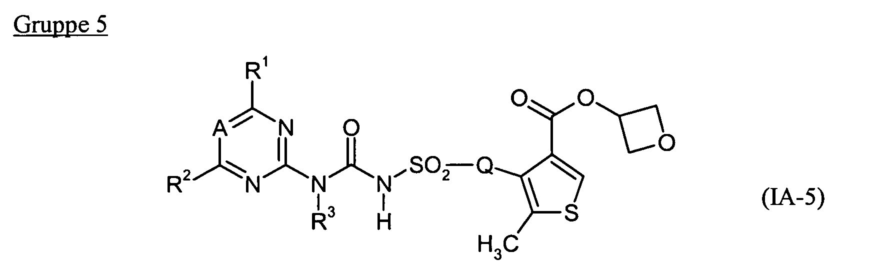 A, Q, R¹, R² und R³ haben hierbei beispielhaft die oben in Gruppe 1 angegebene Bedeutung. 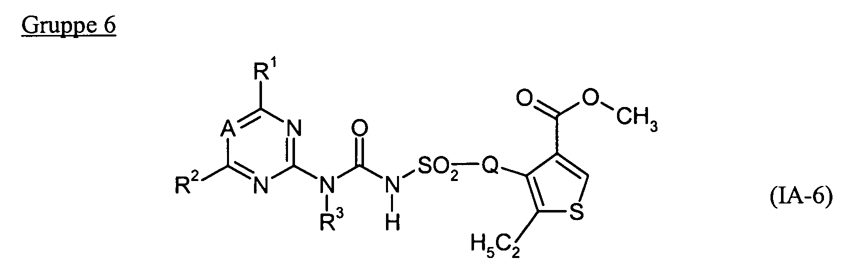 A, Q, R¹, R² und R³ haben hierbei beispielhaft die oben in Gruppe 1 angegebene Bedeutung. 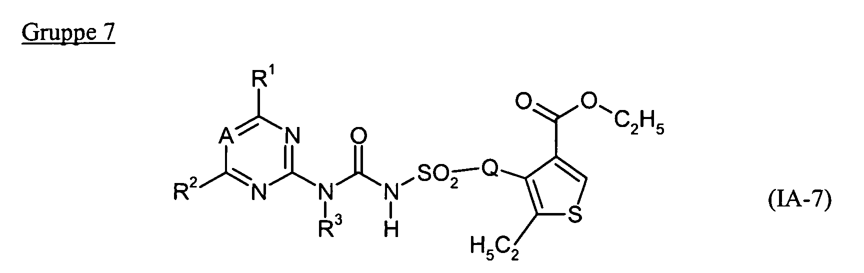 A, Q, R¹, R² und R³ haben hierbei beispielhaft die oben in Gruppe 1 angegebene Bedeutung. 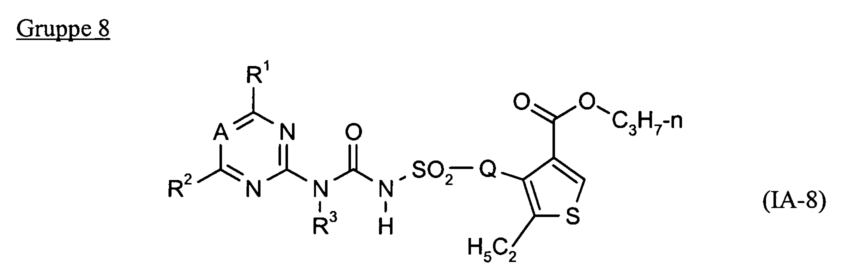 A, Q, R¹, R² und R³ haben hierbei beispielhaft die oben in Gruppe 1 angegebene Bedeutung. 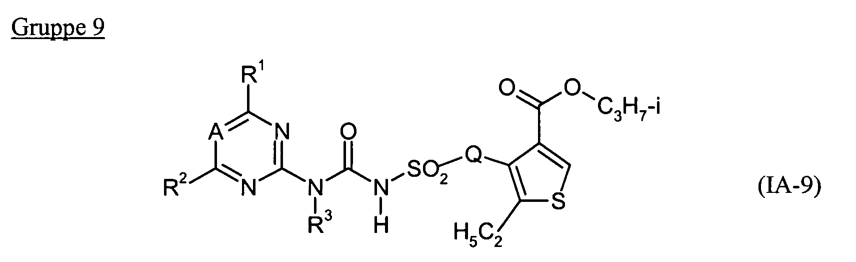 A, Q, R¹, R² und R³ haben hierbei beispielhaft die oben in Gruppe 1 angegebene Bedeutung. 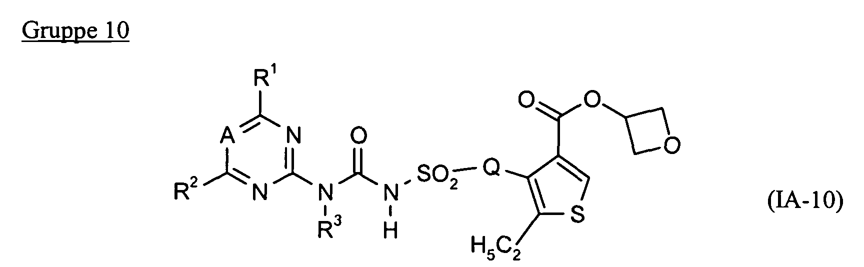 A, Q, R¹, R² und R³ haben hierbei beispielhaft die oben in Gruppe 1 angegebene Bedeutung. 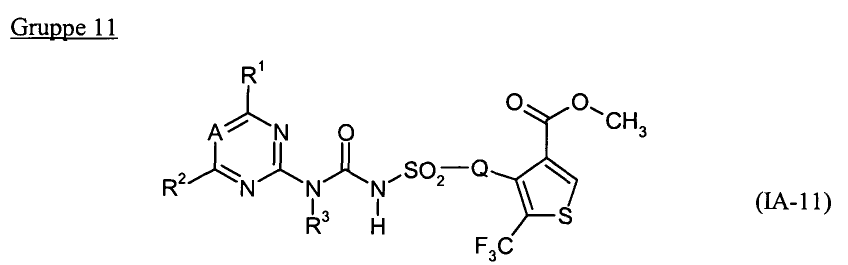 A, Q, R¹, R² und R³ haben hierbei beispielhaft die oben in Gruppe 1 angegebene Bedeutung. 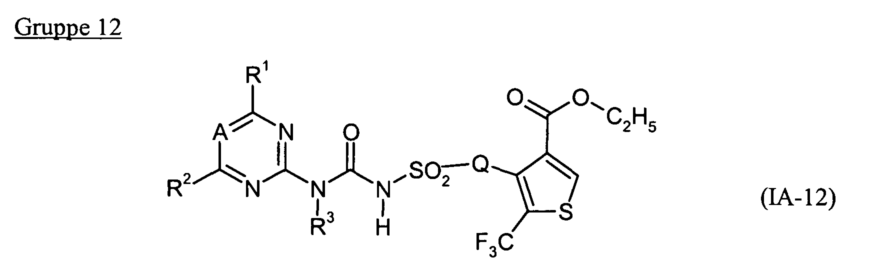 A, Q, R¹, R² und R³ haben hierbei beispielhaft die oben in Gruppe 1 angegebene Bedeutung. 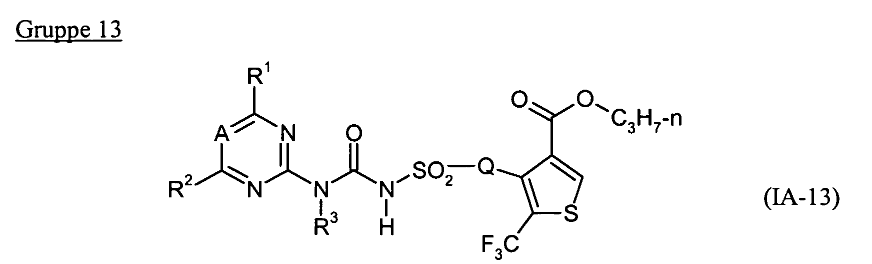 A, Q, R¹, R² und R³ haben hierbei beispielhaft die oben in Gruppe 1 angegebene Bedeutung. 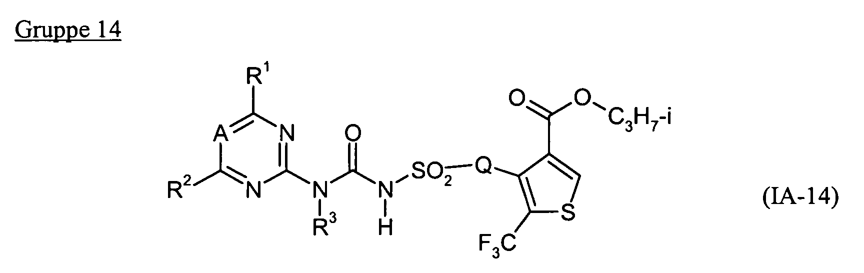 A, Q, R¹, R² und R³ haben hierbei beispielhaft die oben in Gruppe 1 angegebene Bedeutung. 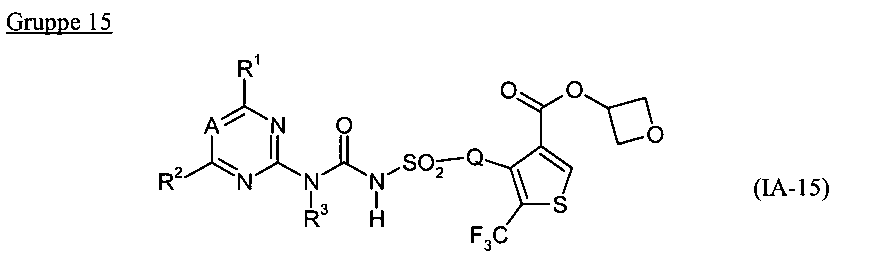 A, Q, R¹, R² und R³ haben hierbei beispielhaft die oben in Gruppe 1 angegebene Bedeutung. 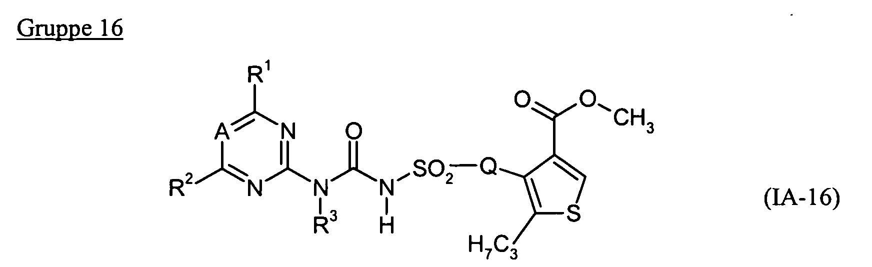 A, Q, R¹, R² und R³ haben hierbei beispielhaft die oben in Gruppe 1 angegebene Bedeutung. 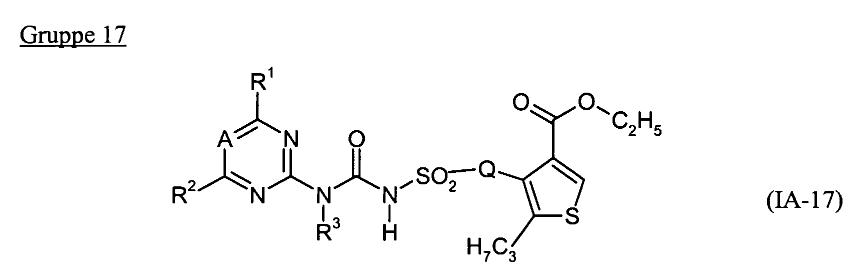 A, Q, R¹, R² und R³ haben hierbei beispielhaft die oben in Gruppe 1 angegebene Bedeutung. 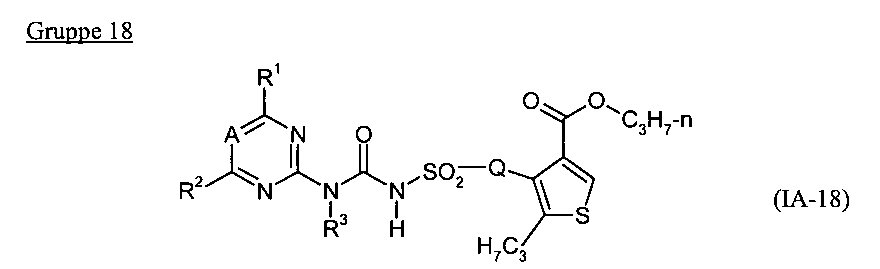 A, Q, R¹, R² und R³ haben hierbei beispielhaft die oben in Gruppe 1 angegebene Bedeutung. 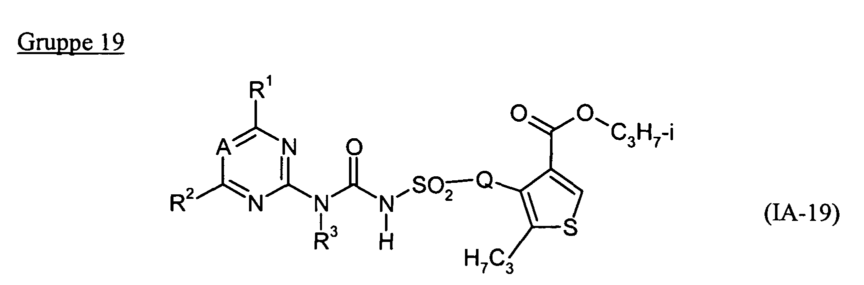 A, Q, R¹, R² und R³ haben hierbei beispielhaft die oben in Gruppe 1 angegebene Bedeutung. 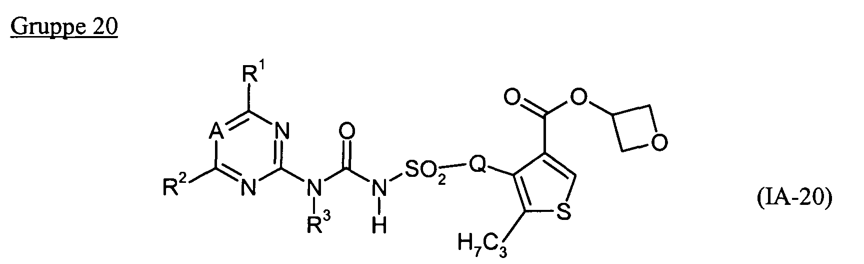 A, Q, R¹, R² und R³ haben hierbei beispielhaft die oben in Gruppe 1 angegebene Bedeutung. 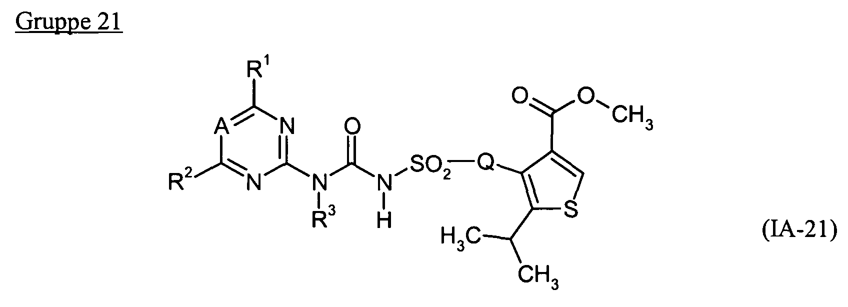 A, Q, R¹, R² und R³ haben hierbei beispielhaft die oben in Gruppe 1 angegebene Bedeutung. 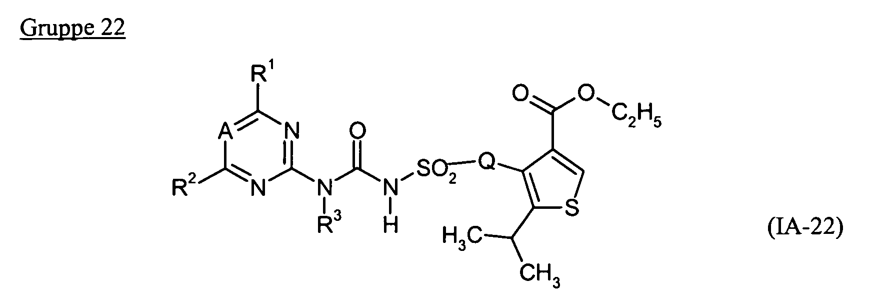 A, Q, R¹, R² und R³ haben hierbei beispielhaft die oben in Gruppe 1 angegebene Bedeutung. 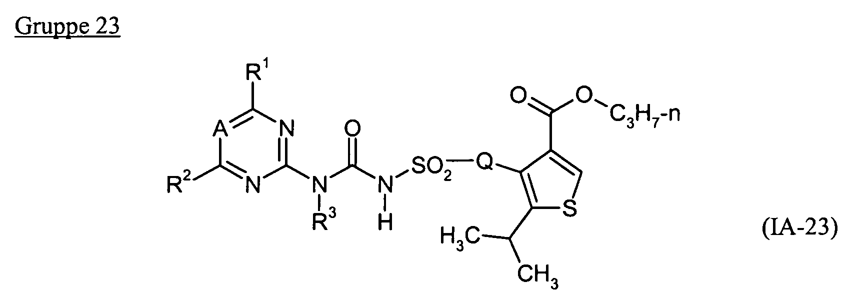 A, Q, R¹, R² und R³ haben hierbei beispielhaft die oben in Gruppe 1 angegebene Bedeutung. 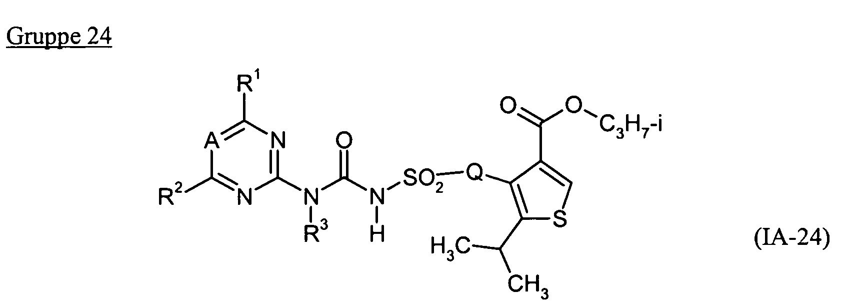 A, Q, R¹, R² und R³ haben hierbei beispielhaft die oben in Gruppe 1 angegebene Bedeutung. 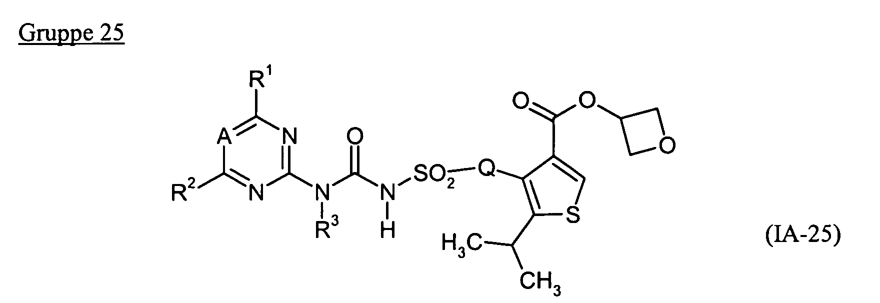 A, Q, R¹, R² und R³ haben hierbei beispielhaft die oben in Gruppe 1 angegebene Bedeutung.

Die neuen substituierten Thienyl(amino)sulfonylharnstoffe der allgemeinen Formel (I) zeichnen sich durch starke herbizide Wirksamkeit aus.

Man erhält die neuen substituierten Thienyl(amino)sulfonylharnstoffe der allgemeinen Formel (I), wenn man

### (a) Aminoazine der allgemeinen Formel (II)

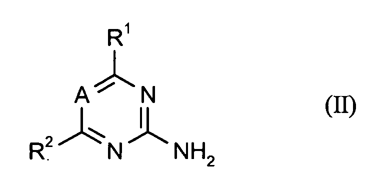

in welcher
A, R¹ und R² die oben angegebene Bedeutung haben,
mit Thienyl(amino)sulfonylisocyanaten der allgemeinen Formel (III) 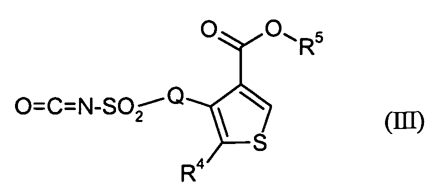
in welcher
Q, R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
oder wenn man

### (b) substituierte Aminoazine der allgemeinen Formel (IV)

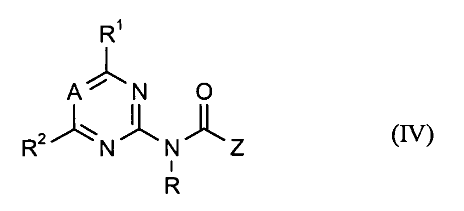

in welcher
- A, R¹ und R²: die oben angegebene Bedeutung haben,
- Z: für Halogen, Alkoxy oder Aryloxy steht und
- R: die oben für R³ angegebene Bedeutung hat oder für die Gruppierung -C(O)-Z steht,
mit Thiophenderivaten der allgemeinen Formel (V) 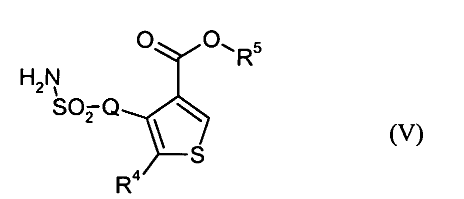 in welcher
Q, R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
oder wenn man

### (c) Aminoazine der allgemeinen Formel (II)

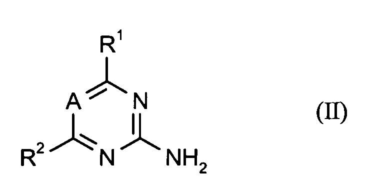

in welcher
A, R¹ und R² die oben angegebene Bedeutung haben,
mit Thiophenderivaten der allgemeinen Formel (VI) 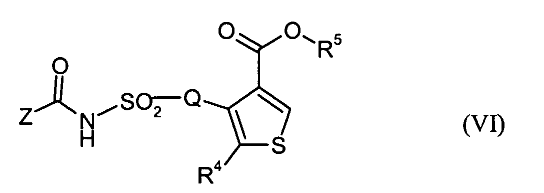
in welcher
- Q, R⁴ und R⁵: die oben angegebene Bedeutung haben und
- Z: für Halogen, Alkoxy oder Aryloxy steht,

gegebenenfalls in Gegenwart eines Reaktionshilfsmittels und gegebenenfalls in Gegenwart eines Verdünnungsmittels umsetzt,
oder wenn man

### (d) Aminoazine der allgemeinen Formel (II)

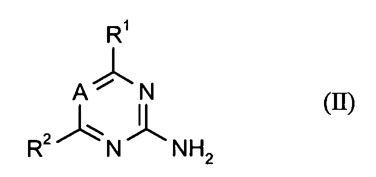

in welcher
A, R¹ und R² die oben angegebene Bedeutung haben,
mit Chlorsulfonylisocyanat gegebenenfalls in Gegenwart eines Verdünnungsmittels umsetzt und die hierbei gebildeten Chlorsulfonylaminocarbonylamino-azine der allgemeinen Formel (VII) 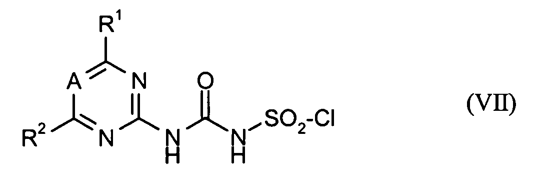
in welcher
A, R¹ und R² die oben angegebene Bedeutung haben,
- nach Zwischenisolierung oder "in situ" -
mit substituierten Aminothiophenen der allgemeinen Formel (VIII) 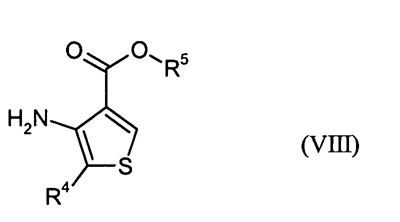
in welcher
R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
und gegebenenfalls die nach den Verfahren (a), (b), (c) oder (d) erhaltenen Verbindungen der Formel (I) nach üblichen Methoden in Salze überführt.

Verwendet man beispielsweise 2-Amino-4-methoxy-6-methyl-pyrimidin und 4-Ethoxycarbonyl-2-trifluormethyl-thien-3-yl-sulfonylisocyanat als Ausgangsstoffe, so kann der Reaktionsablauf beim erfindungsgemäßen Verfahren (a) durch das folgende Formelschema skizziert werden:

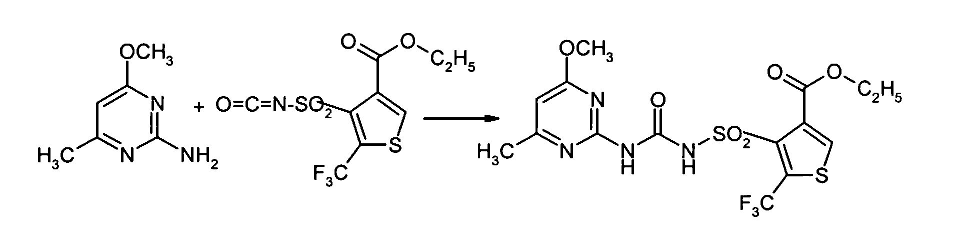

Verwendet man beispielsweise 2-Methoxycarbonylamino-4-methoxy-6-trifluormethyl-1,3,5-triazin und 2-Ethyl-4-i-propoxycarbonyl-thiophen-3-sulfonamid als Ausgangsstoffe, so kann der Reaktionsablauf beim erfindungsgemäßen Verfahren (b) durch das folgende Formelschema skizziert werden:

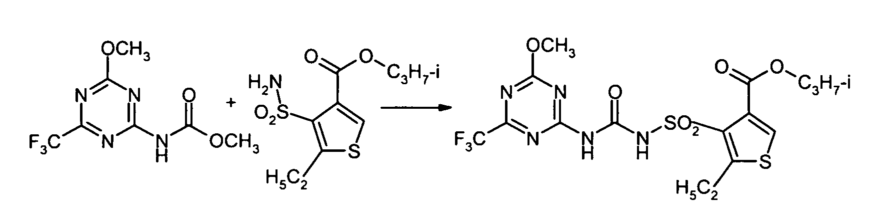

Verwendet man beispielsweise 2-Amino-4-chlor-6-methoxy-pyrimidin und N-(4-Ethoxycarbonyl-2-methyl-thien-3-yl-sulfonyl)-O-phenyl-urethan als Ausgangsstoffe, so kann der Reaktionsablauf beim erfindungsgemäßen Verfahren (c) durch das folgende Formelschema skizziert werden:

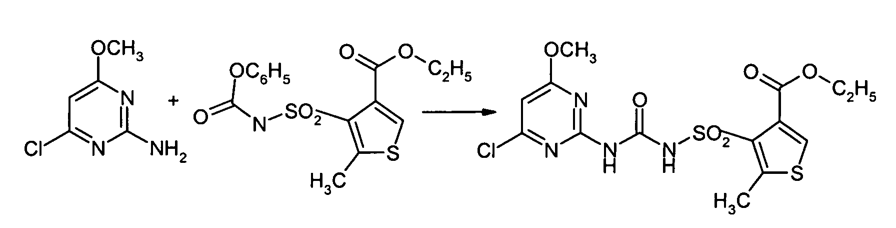

Verwendet man beispielsweise 2-Amino-4-methoxy-6-trifluormethyl-pyrimidin, Chlorsulfonylisocyanat und 3-Amino-2-ethyl-thiophen-4-carbonsäure-ethylester als Ausgangsstoffe, so kann der Reaktionsablauf beim erfindungsgemäßen Verfahren (d) durch das folgende Formelschema skizziert werden: 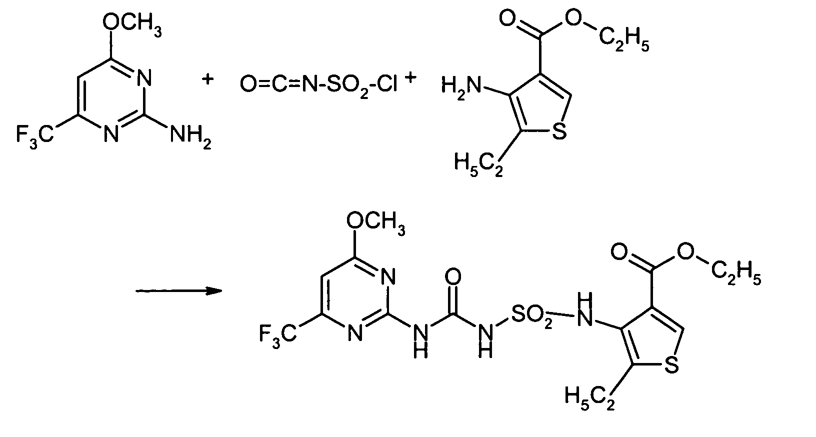 Die bei den erfindungsgemäßen Verfahren (a), (c) und (d) zur Herstellung der Verbindungen der allgemeinen Formel (I) als Ausgangsstoffe zu verwendenden Aminoazine sind durch die Formel (II) allgemein definiert. In der Formel (II) haben A, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹ und R² angegeben wurden.

Die Aminoazine der Formel (II) sind bekannte, zum Teil im Handel erhältliche Synthesechemikalien.

Die beim erfindungsgemäßen Verfahren (a) weiter als Ausgangsstoffe zu verwendenden Thienyl(amino)sulfonylisocyanate sind durch die Formel (III) allgemein definiert. In der Formel (III) haben Q, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴ und R⁵ angegeben wurden.

Die Ausgangsstoffe der Formel (III) sind bekannt und/oder können nach an sich bekannten Verfahren hergestellt werden (vgl. EP 30142 / US-A-4481029 / US-A-4599103 / US-A-4701535).

Man erhält die Thienyl(amino)sulfonylisocyanate der Formel (III), wenn man Thiophenderivate der allgemeinen Formel (V) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]-octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die beim erfindungsgemäßen Verfahren (b) zur Herstellung der Verbindungen der Formel (I) als Ausgangsstoffe zu verwendenden substituierten Aminoazine sind durch die Formel (IV) allgemein definiert. In der Formel (IV) haben A, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹ und R² angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (IV) sind bekannt und/oder können nach an sich bekannten Verfahren hergestellt werden (vgl. US 4690707, DE 19501174).

Die beim erfindungsgemäßen Verfahren (b) weiter als Ausgangsstoffe zu verwendenden Thiophenderivate sind durch die Formel (V) allgemein definiert. In der Formel (V) haben Q, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴ und R⁵ angegeben wurden.

Die Ausgangsstoffe der Formel (V) sind bekannt und/oder können nach an sich bekannten Verfahren hergestellt werden (vgl. EP 30142 / US-A-4481029 / US-A-4599103 / US-A-4701535, Herstellungsbeispiele).

Die beim erfindungsgemäßen Verfahren (c) zur Herstellung der Verbindungen der Formel (I) als Ausgangsstoffe zu verwendenden substituierten Thiophenderivate sind durch die Formel (VI) allgemein definiert. In der Formel (VI) haben Q, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴ und R⁵ angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (VI) sind bekannt und/oder können nach an sich bekannten Verfahren hergestellt werden.

Die beim erfindungsgemäßen Verfahren (d) zur Herstellung von Verbindungen der allgemeinen Formel (I) als Ausgangsstoffe zu verwendenden substituierten Aminothiophene sind durch die Formel (VIII) allgemein definiert. In der allgemeinen Formel (VIII) haben R⁴ und R⁵ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R⁴ und R⁵ angegeben worden sind.

Die Ausgangsstoffe der allgemeinen Formel (VIII) sind bekannt und/oder können nach an sich bekannten Verfahren hergestellt werden (vgl. Aust. J. Chem. 48 (1995), 1907-1916).

Als Verdünnungsmittel zur Durchführung der erfindungsgemäßen Verfahren (a), (b), (c) und (d) kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, sowie Sulfoxide, wie Dimethylsulfoxid.

Die erfindungsgemäßen Verfahren (a), (b), (c) und (d) werden vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetall- -hydride, -hydroxide, -amide, -alkoholate,-acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a), (b), (c) und (d) in einem größeren Bereich variiert werden. Im allgemeinen arbeitet man bei Temperaturen zwischen -20°C und +150°C, vorzugsweise zwischen -10°C und +120°C.

Die erfindungsgemäßen Verfahren (a), (b), (c) und (d) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren (a), (b), (c) und (d) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Fentrazamide, Flamprop(-isopropyl), Flamprop(-isopropyl-L), Flamprop(-methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron(-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellunsgsbeispiele:

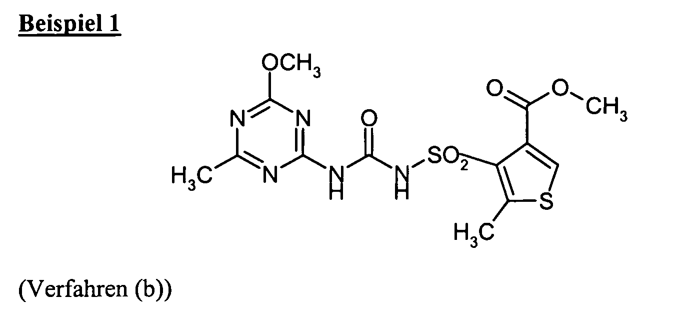

0,75 g (2,9 mMol) 2-Phenoxycarbonylamino-4-methoxy-6-methyl-1,3,5-triazin werden in 40 ml Acetonitril gelöst und nacheinander mit 0,75 g (3,2 mMol) 2-Methyl-3-sulfamoyl-thiophen-4-carbonsäure-methylester und 0,49 g (3,2 mMol) Diazabicycloundecen (DBU) versetzt. Die Reaktionsmischung wird 12 Stunden bei Raumtemperatur (ca. 20°C) gerührt und anschließend im Wasserstrahlvakuum eingeengt. Der Rückstand wird in Methylenchlorid aufgenommen, mit 2N-Salzsäure und mit Wasser gewaschen, mit Magnesiumsulfat getrocknet und filtriert. Das Filtrat wird im Wasserstrahlvakuum eingeengt, der Rückstand mit Diethylether digeriert und das kristallin angefallene Produkt durch Absaugen isoliert.

Man erhält 0,60 g (52% der Theorie) N-(4-Methoxy-6-methyl-1,3,5-triazin-2-yl)-N'-(4-methoxycarbonyl-2-methyl-thien-3-yl-sulfonyl)-harnstoff vom Schmelzpunkt 195°C. 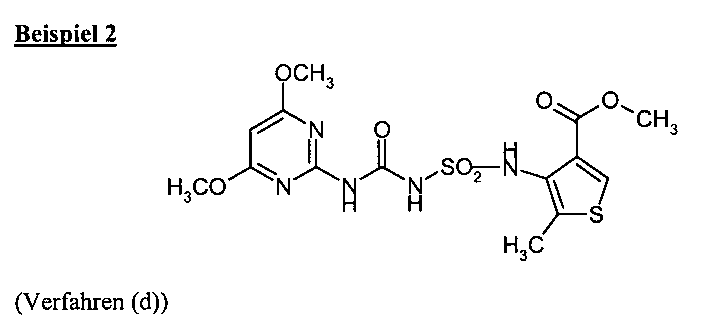 1,05 g (7,5 mMol) Chlorsulfonylisocyanat werden in 75 ml Methylenchlorid vorgelegt. Nach Abkühlen auf -10°C wird zu dieser Mischung unter Rühren eine Lösung von 1,16 g (7,5 mMol) 2-Amino-4,6-dimethoxy-pyrimidin in 30 ml Methylenchlorid tropfenweise gegeben und die Mischung wird 30 Minuten bei -10°C gerührt. Dann wird bei 0°C eine Lösung von 1,28 g (7,5 mMol) 3-Amino-2-methyl-thiophen-4-carbonsäure-methylester und 0,75 g (7,5 mMol) Triethylamin in 50 ml Methylenchlorid tropfenweise dazu gegeben und die Reaktionsmischung wird 12 Stunden bei Raumtemperatur (ca. 20°C) gerührt. Anschließend werden 100 ml Wasser und 100 ml 2N-Salzsäure dazu gegeben, die organische Phase abgetrennt, mit 50 ml Wasser gewaschen, mit Magnesiumsulfat getrocknet und filtriert. Das Filtrat wird im Wasserstrahlvakuum eingeengt und der Rückstand aus Ethanol kristallisiert.

Man erhält 2,1 g (66% der Theorie) N-(4,6-Dimethoxy-pyrimidin-2-yl)-N'-(4-methoxycarbonyl-2-methyl-thien-3-yl-amino-sulfonyl)-harnstoff vom Schmelzpunkt 174°C.

Analog zu den Beispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

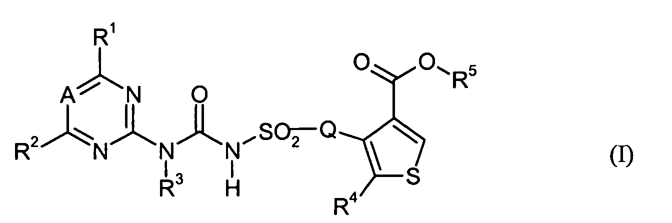

**Tabelle 1:**

| Beispiele für die Verbindungen der Formel (I) | | | | | | | | |
|---|---|---|---|---|---|---|---|---|
| Bsp.-Nr. | A | Q | R¹ | R² | R³ | R⁴ | R⁵ | Schmelzpunkt (°C) |
| 3 | CH | NH | OCH₃ | OCH₃ | H | n-C₃H₇ | C₂H₅ | 140 |
| 4 | CH | NH | OCH₃ | OCH₃ | H | i-C₃H₇ | C₂H₅ | 154 |
| 5 | CH | NH | OCH₃ | OCH₃ | H | C₂H₅ | CH₃ | 195 |
| 6 | CH | - | OCH₃ | OCH₃ | H | CH₃ | CH₃ | 194 |

### Ausgangsstoffe der Formel (V):

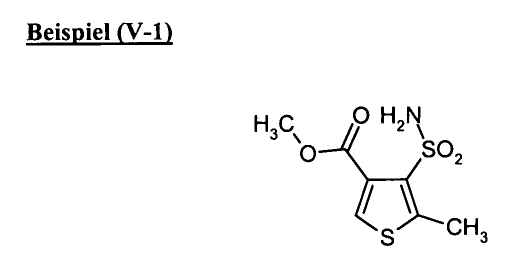

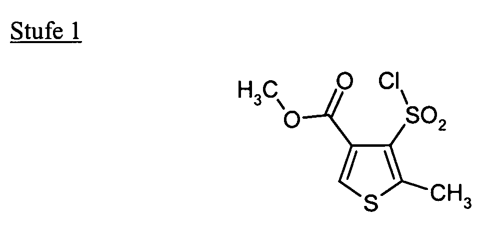

Eine Lösung von 19,9 g (0,29 Mol) Natriumnitrit in 60 ml Wasser wird bei 0°C bis 5°C tropfenweise unter Rühren zu einer Lösung von 42,7 g (0,25 Mol) 3-Amino-2-methyl-thiophen-4-carbonsäure-methylester in 75 ml 10%iger wässriger Salzsäure gegeben. Die Reaktionsmischung wird 60 Minuten bei 0°C bis 5°C gerührt. Anschließend wird der Nitrit-Überschuß mit Amidosulfonsäure beseitigt. Die Mischung wird dann bei 0°C bis 5°C tropfenweise unter Rühren zu einer Lösung von 35 g (0,55 Mol) Schwefeldioxid in 300 ml Methylenchlorid gegeben. Nach Zugabe von 1,5 g Kupfer(I)-chlorid und 1,5 g Dodecyl-trimethylammonium-bromid wird die Reaktionsmischung 60 Minuten bei 40°C und weiter 12 Stunden bei 20°C gerührt. Anschließend werden 18 ml 35%ige wässrige Salzsäure dazu gegeben, die Mischung 4 Stunden bei 20°C gerührt und dann die Phasen getrennt. Die wässrige Phase wird mit Methylenchlorid nachextrahiert, die vereinigten organischen Phasen mit Wasser gewaschen, mit Magnesiumsulfat getrocknet und filtriert. Das Filtrat wird im Wasserstrahlvakuum eingeengt und der Rückstand aus Hexan kristallisiert.

Man erhält 51,7 g (81% der Theorie) 4-Methoxycarbonyl-2-methyl-thiophen-3-sulfonsäurechlorid.

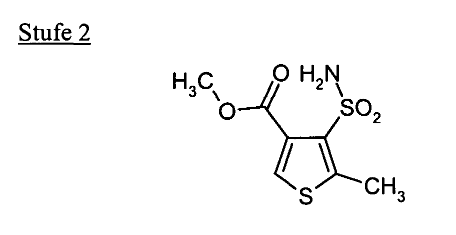

Eine Mischung aus 45 g (177 mMol) 4-Methoxycarbonyl-2-methyl-thiophen-3-sulfonsäurechlorid, 34 g (354 mMol) Ammoniumcarbonat und 400 ml Methylenchlorid wird 12 Stunden bei Raumtemperatur (ca. 20°C) gerührt. Nach Filtration wird vom Filtrat das Lösungsmittel im Wasserstrahlvakuum abdestilliert, der Rückstand mit Diethylether digeriert und das kristalline Produkt durch Absaugen isoliert.

Man erhält 21,5 g (52% der Theorie) 4-Methoxycarbonyl-2-methyl-thiophen-3-sulfonamid.

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 2, 3, 4, 5 und 6 bei teilweise guter Verträglichkeit gegenüber Kulturpflanzen, wie z.B. Baumwolle, Mais und Soja, sehr starke Wirkung gegen Unkräuter.

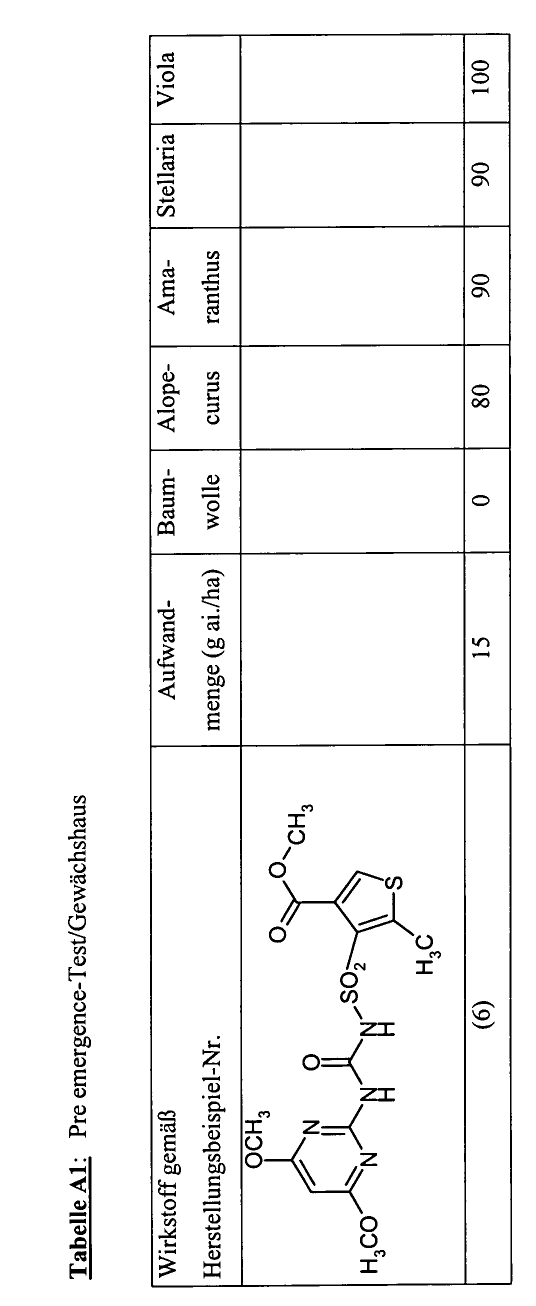

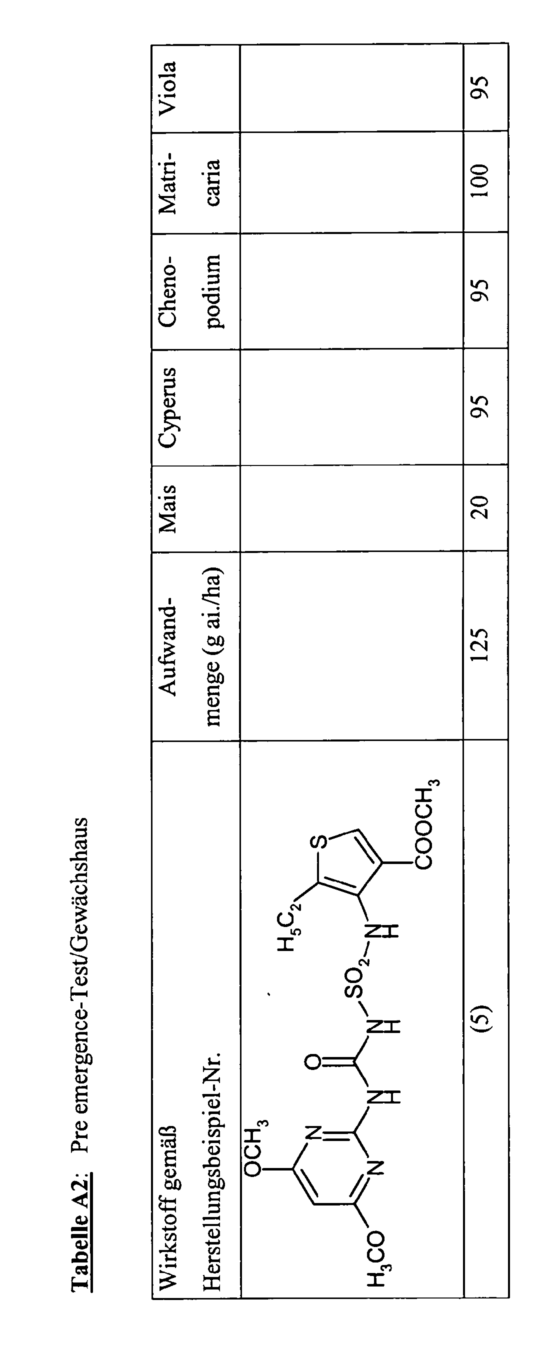

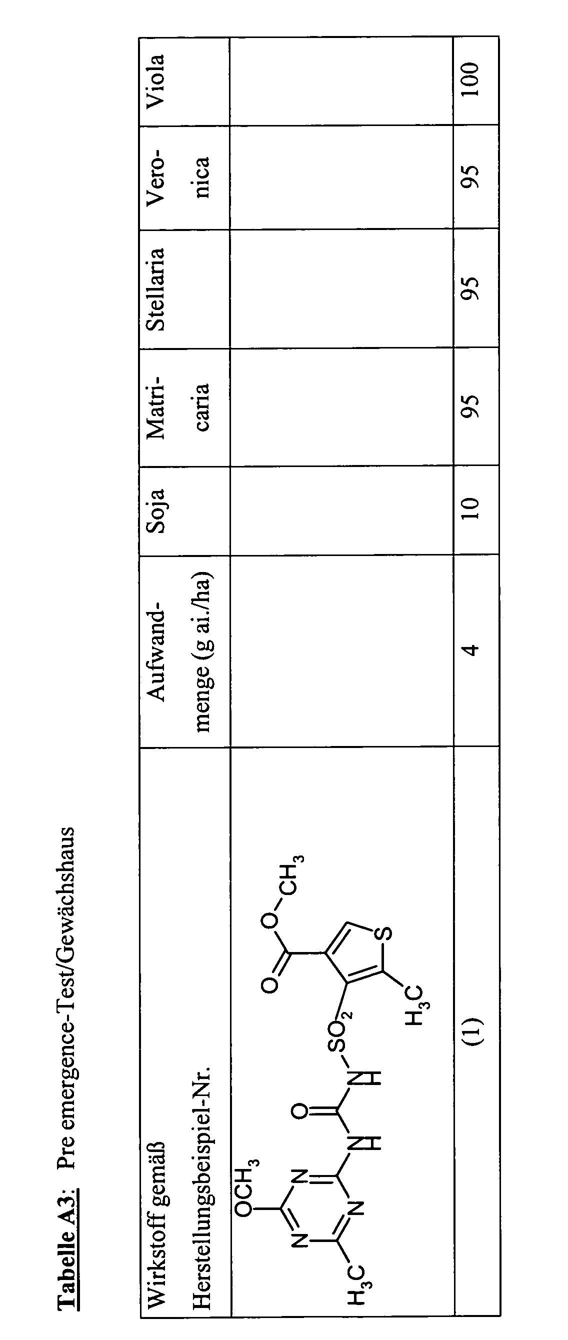

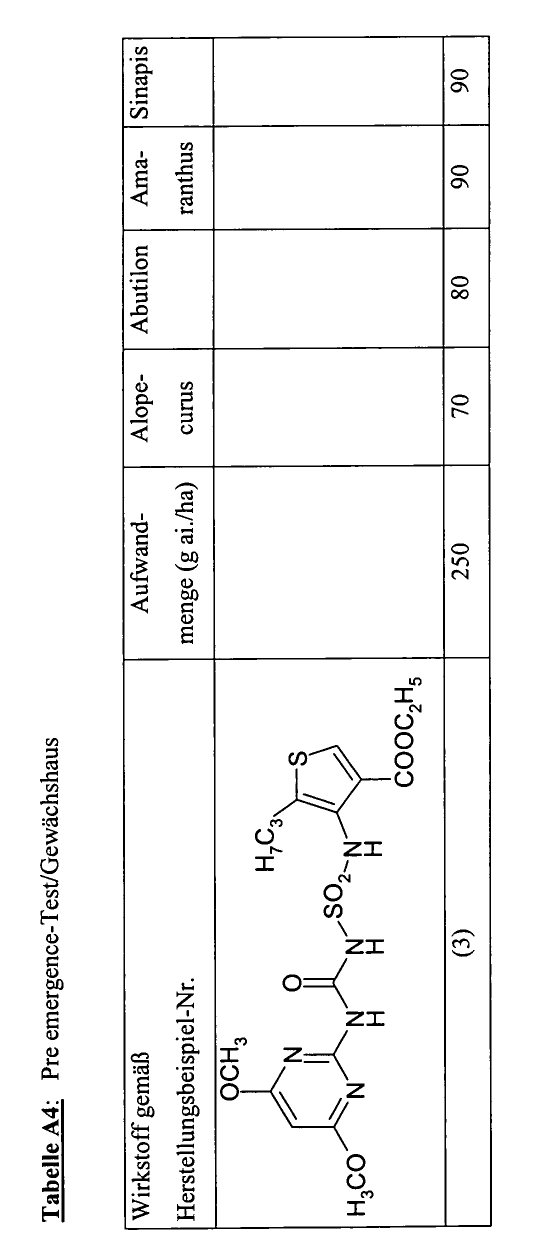

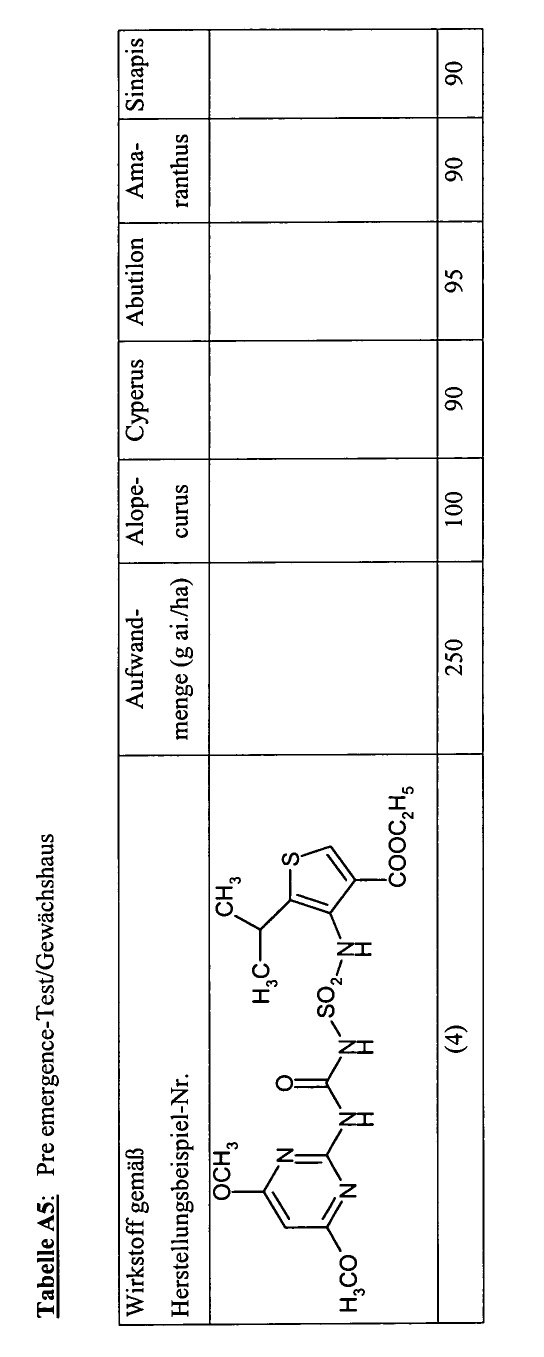

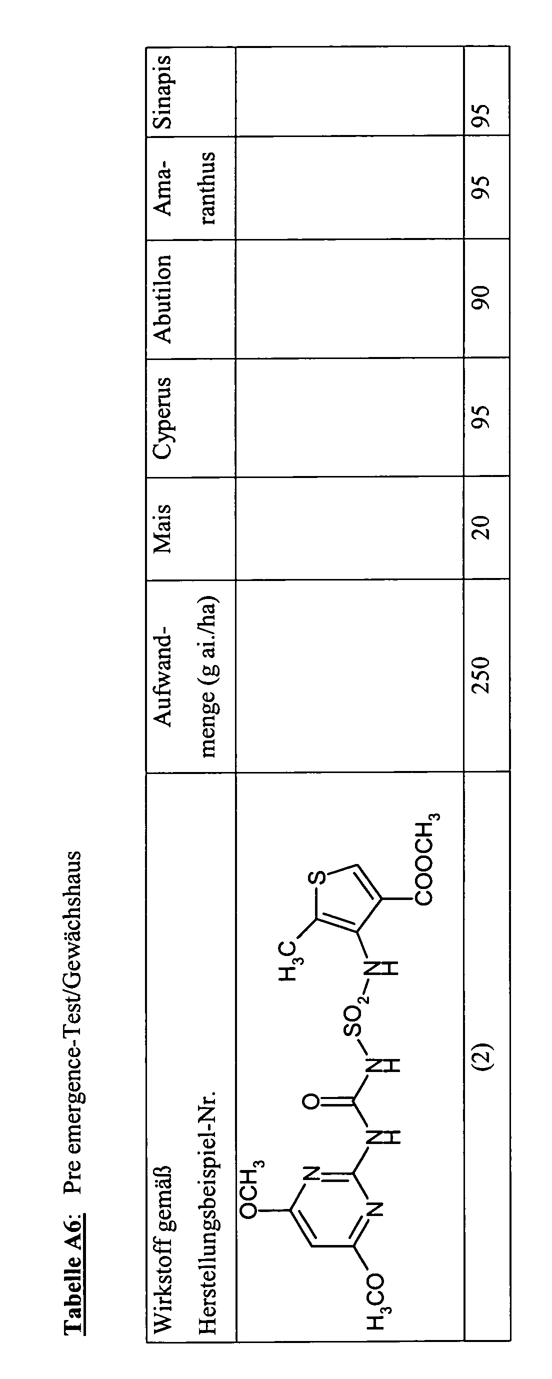

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 2, 5 und 6 bei teilweise guter Verträglichkeit gegenüber Kulturpflanzen, wie z.B. Gerste und Weizen, sehr starke Wirkung gegen Unkräuter.

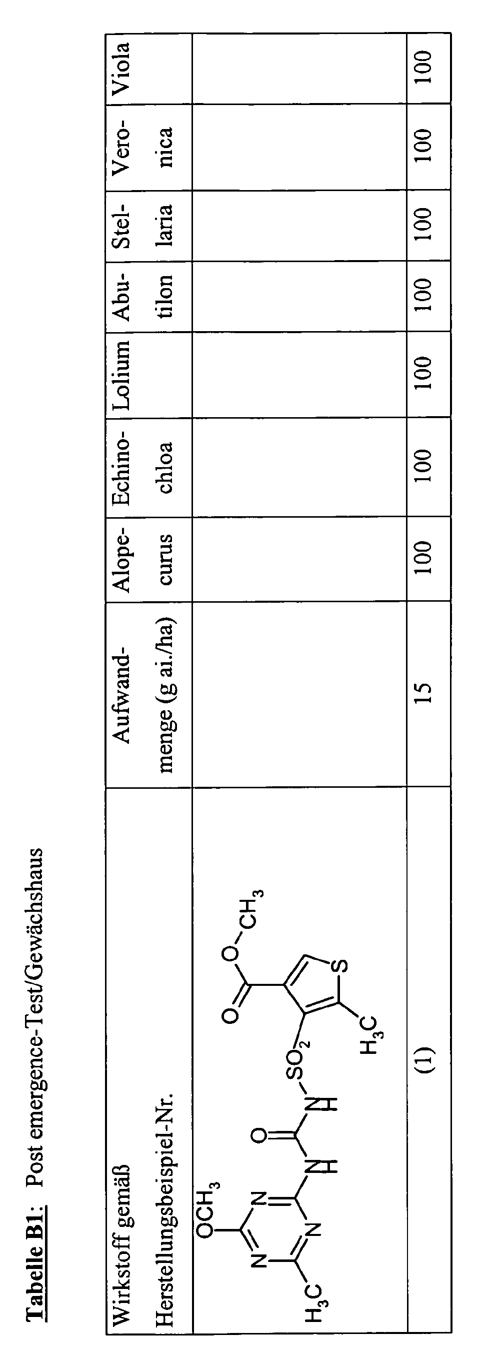

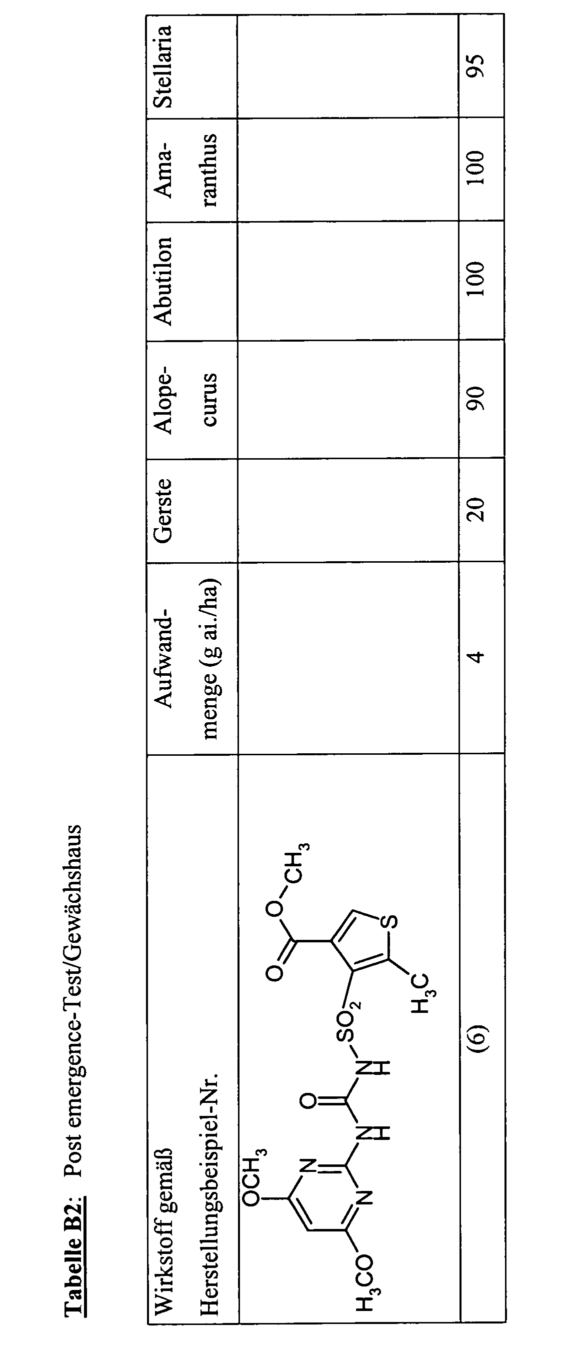

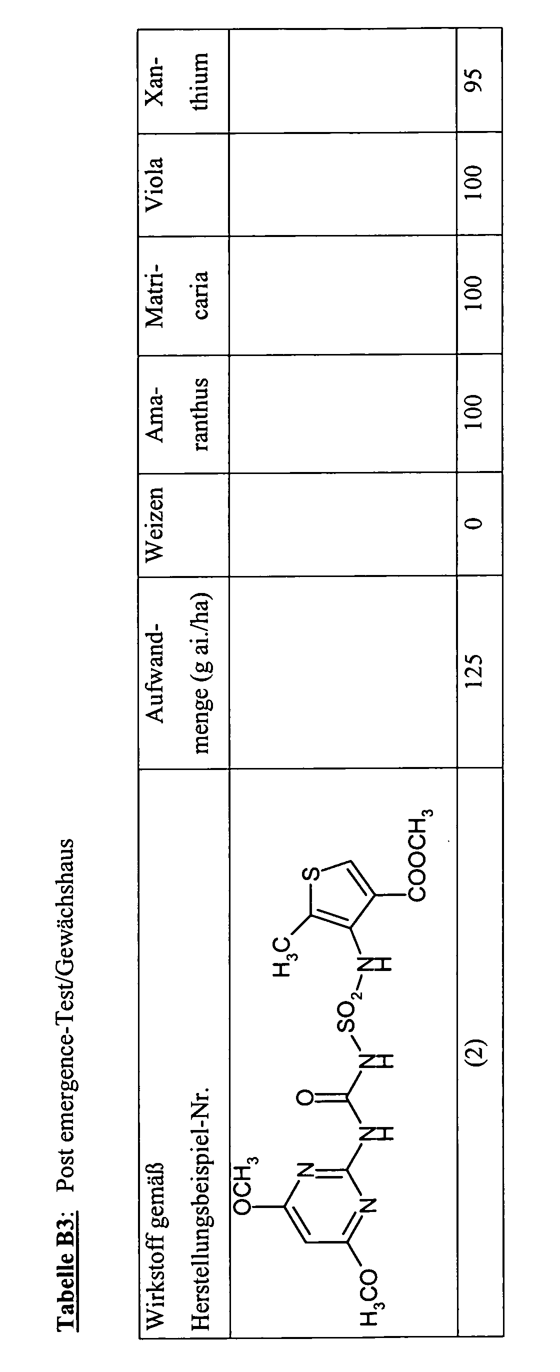

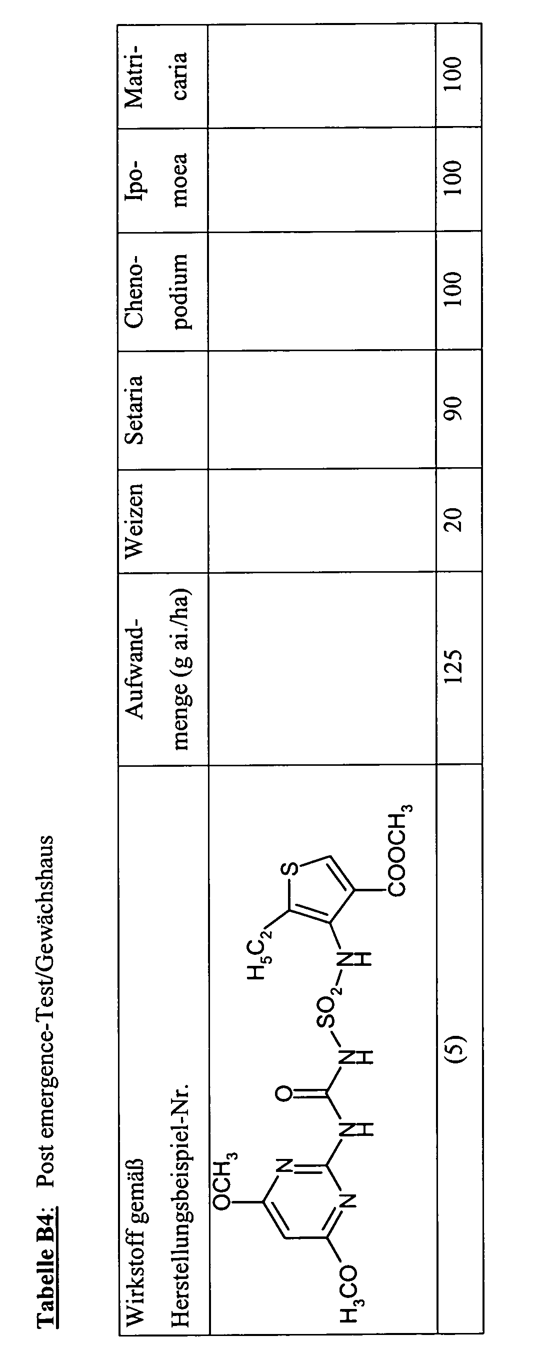

## Patentansprüche

1. Verbindungen der allgemeinen Formel (I) 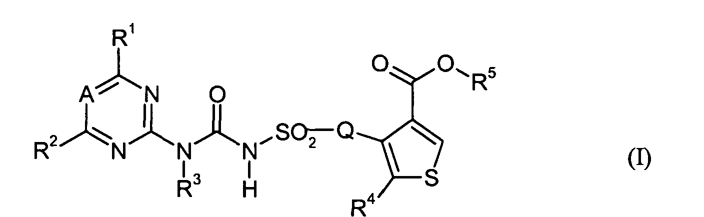 in welcher
A für eine CH-Gruppierung steht,
Q für eine Einfachbindung oder für NH steht,
R¹ für Wasserstoff, Halogen oder jeweils gegebenenfalls substituiertes Alkyl, Alkoxy, Alkylthio, Alkylamino, Dialkylamino, Aryloxy oder Heterocyclyloxy steht,
R² für Wasserstoff, Halogen oder jeweils gegebenenfalls substituiertes Alkyl, Alkoxy, Alkylthio, Alkylamino, Dialkylamino, Aryloxy oder Heterocyclyloxy steht,
R³ für Wasserstoff oder gegebenenfalls substituiertes Alkyl steht,
R⁴ für Halogen oder gegebenenfalls substituiertes Alkyl steht und - für den Fall, daß Q für NH steht - auch für Wasserstoff steht, und
R⁵ für Wasserstoff oder jeweils gegebenenfalls substituiertes Alkyl, Alkenyl, Alkinyl, Cycloalkyl, Cycloalkylalkyl oder Heterocyclyl steht, sowie Salze von Verbindungen der Formel (I).

2. Verbindungen gemäß Anspruch 1, **dadurch gekennzeichnet, daß**
R¹ für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
R² für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
R³ für Wasserstoff oder gegebenenfalls durch C₁-C₄-Alkoxy, C₁-C₄- Alkyl-carbonyl oder C₁-C₄-Alkoxy-carbonyl substituiertes Alkyl mit 1 bis 4 Kohlenstoffatomen steht,
R⁴ für gegebenenfalls durch Cyano, Halogen oder C₁-C₄-Alkoxy substituiertes Alkyl mit 1 bis 6 Kohlenstoffatomen steht und - für den Fall, daß Q für NH steht - auch für Wasserstoff steht, und
R⁵ für Wasserstoff, für gegebenenfalls durch Cyano, Halogen oder C₁-C₄-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Cyano, Halogen, C₁-C₄-Alkyl oder C₁-C₄-Alkoxy substituiertes Oxetanyl, Furyl oder Tetrahydrofuryl steht.

3. Verbindungen gemäß Anspruch 1, **dadurch gekennzeichnet, daß**
R¹ für Wasserstoff, Fluor, Chlor, Brom, Iod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom, oder für jeweils gegebenenfalls durch Cyano, Fluor, Chlor, Methoxy oder Ethoxy substituiertes Methyl, Ethyl, n-oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R³ für Wasserstoff oder für jeweils gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
R⁴ für jeweils gegebenenfalls durch Cyano, Fluor, Chlor, Methoxy oder Ethoxy substituiertes Methyl, Ethyl, n- oder i-Propyl, n-, i-, s- oder t-Butyl steht, und
R⁵ für Wasserstoff, für jeweils gegebenenfalls durch Cyano, Fluor, Chlor, Methoxy, Ethoxy, n- oder i-Propoxy substituiertes Methyl, Ethyl, n-oder i-Propyl, n-, i-, s- oder t-Butyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht.

4. Verbindungen gemäß Anspruch 1, **dadurch gekennzeichnet, daß**
R¹ für Wasserstoff, Fluor, Chlor, Brom, für jeweils gegebenenfalls durch Fluor, Chlor, Methoxy oder Ethoxy substituiertes Methyl, Ethyl, Methoxy, Ethoxy, Methylthio, Ethylthio, Methylamino, Ethylamino, oder für Dimethylamino steht,
R² für Fluor, Chlor, Brom, für jeweils gegebenenfalls durch Fluor, Chlor, Methoxy oder Ethoxy substituiertes Methyl, Ethyl, Methoxy, Ethoxy, Methylthio, Ethylthio, Methylamino oder Ethylamino, oder für Dimethylamino steht,
R³ für Wasserstoff oder Methyl steht,
R⁴ für jeweils gegebenenfalls durch Fluor oder Chlor substituiertes Methyl, Ethyl, n- oder i-Propyl steht, und
R⁵ für jeweils gegebenenfalls durch Fluor, Chlor, Methoxy oder Ethoxy substituiertes Methyl, Ethyl, n- oder i-Propyl, oder für jeweils gegebenenfalls durch Fluor oder Chlor substituiertes Propenyl oder Propinyl steht.

5. Natrium-, Kalium-, Magnesium-, Calcium-, Ammonium-, C₁-C₄-Alkylammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen gemäß einem der Ansprüche 1 bis 4.

6. Verfahren zum Herstellen von Verbindungen gemäß einem der Ansprüche 1 bis 5, **dadurch gekennzeichnet, daß**
(a) Aminoazine der allgemeinen Formel (II) 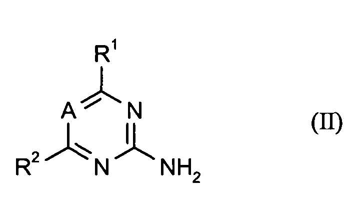 in welcher
A, R¹ und R² in die einem der Ansprüche 1 bis 4 angegebene Bedeutung haben,
mit Thienyl(amino)sulfonylisocyanaten der allgemeinen Formel (III) 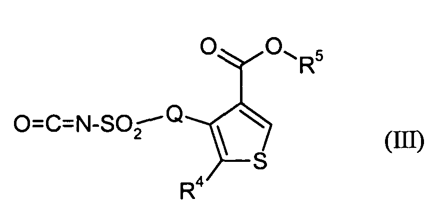 in welcher
Q, R⁴ und R⁵ die in einem der Ansprüche 1 bis 4 angegebene Bedeutung haben,
gegebenenfalls in Gegenwart eines Reaktionshilfsmittels und gegebenenfalls in Gegenwart eines Verdünnungsmittels umgesetzt werden,
oder daß
(b) substituierte Aminoazine der allgemeinen Formel (IV) 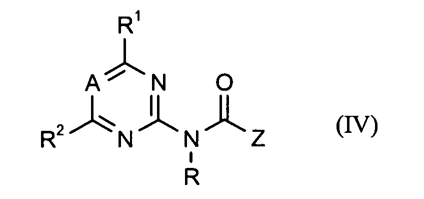 in welcher
A, R¹ und R² die in einem der Ansprüche 1 bis 4 angegebene Bedeutung haben,
Z für Halogen, Alkoxy oder Aryloxy steht und
R die in einem der Ansprüche 1 bis 4 für R³ angegebene Bedeutung hat oder für die Gruppierung -C(O)-Z steht,
mit Thiophenderivaten der allgemeinen Formel (V) 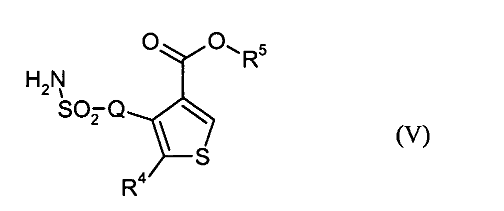 in welcher
Q, R⁴ und R⁵ die in einem der Ansprüche 1 bis 4 angegebene Bedeutung haben,
gegebenenfalls in Gegenwart eines Reaktionshilfsmittels und gegebenenfalls in Gegenwart eines Verdünnungsmittels umgesetzt werden,
oder daß
(c) Aminoazine der allgemeinen Formel (II) 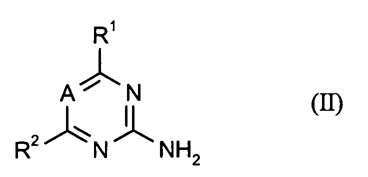 in welcher
A, R¹ und R² die in einem der Ansprüche 1 bis 4 angegebene Bedeutung haben,
mit Thiophenderivaten der allgemeinen Formel (VI) 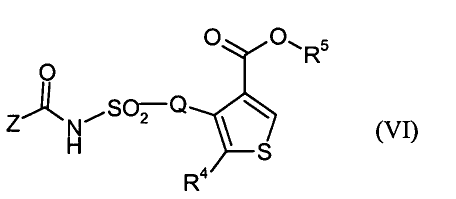
in welcher
Q, R⁴ und R⁵ die in einem der Ansprüche 1 bis 4 angegebene Bedeutung haben und
Z für Halogen, Alkoxy oder Aryloxy steht,
gegebenenfalls in Gegenwart eines Reaktionshilfsmittels und gegebenenfalls in Gegenwart eines Verdünnungsmittels umgesetzt werden,
oder daß
(d) Aminoazine der allgemeinen Formel (II) 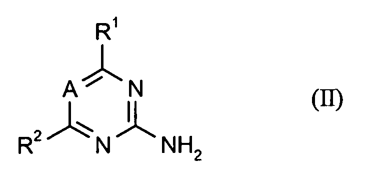 in welcher
A, R¹ und R² die in einem der Ansprüche 1 bis 4 angegebene Bedeutung haben,
mit Chlorsulfonylisocyanat gegebenenfalls in Gegenwart eines Verdünnungsmittels umgesetzt werden und die hierbei gebildeten Chlorsulfonylaminocarbonylamino-azine der allgemeinen Formel (VII) 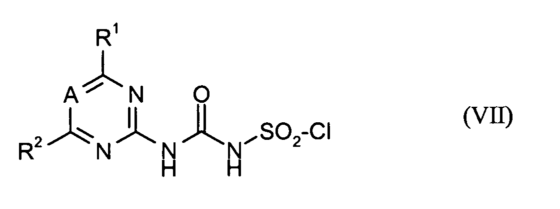
in welcher
A, R¹ und R² die in einem der Ansprüche 1 bis 4 angegebene Bedeutung haben,
- nach Zwischenisolierung oder "in situ" -
mit substituierten Aminothiophenen der allgemeinen Formel (VIII) 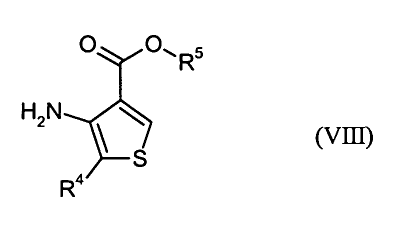
in welcher
R⁴ und R⁵ die in einem der Ansprüche 1 bis 4 angegebene Bedeutung haben,
gegebenenfalls in Gegenwart eines Reaktionshilfsmittels und gegebenenfalls in Gegenwart eines Verdünnungsmittels umgesetzt werden,
und gegebenenfalls die nach den Verfahren (a), (b), (c) oder (d) erhaltenen Verbindungen der Formel (I) nach üblichen Methoden in Salze überführt werden.

7. Verfahren zum Bekämpfen von unerwünschtem Pflanzenwuchs, **dadurch gekennzeichnet, daß** man mindestens eine Verbindung gemäß einem der Ansprüche 1 bis 5 auf unerwünschte Pflanzen und/oder ihren Lebensraum einwirken läßt.

8. Verwendung von mindestens einer Verbindung gemäß einem der Ansprüche 1 bis 5 zum Bekämpfen von unerwünschten Pflanzen.

9. Herbizides Mittel, **gekennzeichnet durch** einen Gehalt an einer Verbindung gemäß einem der Ansprüche 1 bis 5 und üblichen Streckmitteln und/oder oberflächenaktiven Mitteln.
